# FASCICULE DE BREVET EUROPEEN

(11) **EP 2 629 893 B1**
(45) Date de publication et mention de la délivrance du brevet: **11.04.2018**
(21) Numéro de dépôt: 11787718.3
(22) Date de dépôt: 19.10.2011
(51) Int. Cl.: B01L 3/00

(54) **PROCEDE ET DISPOSITIF POUR ISOLER UN PUITS A ECHANTILLON D'UNE CARTE TEST POUR ANALYSE ET CARTE TEST OBTENUE**
VERFAHREN UND VORRICHTUNG ZUM SCHLIESSEN EINER MULDE IN EINER ANALYTISCHEN TESTKARTE UND RESULTIERENDE TESTKARTE
METHOD AND DEVICE OF CLOSING A SAMPLE WELL OF A ANALYTICAL TEST CARD AND TEST CARD RESULTING FROM THE METHOD

(30) Priorité: 22.10.2010 FR 1058699
(43) Date de publication de la demande: 28.08.2013
(73) Titulaire: BIOMERIEUX, 69280 Marcy-L'Etoile (FR)
(72) Inventeur: COLIN, Bruno, F-69280 Marcy L'Etoile (FR); PARIS, Cécile, F-69690 Bessenay (FR)
(74) Mandataire: Thibault, Jean-Marc
(86) Numéro de dépôt international: PCT/FR2011/052437
(87) Numéro de publication internationale: WO 2012/052680

(56) Documents cités:
- WO-A1-2004/011149
- US-A1- 2006 269 451
- US-A1- 2007 059 214
- US-A1- 2007 114 229

## Description

La présente invention concerne le domaine technique général des appareils et machines de test d'échantillons biologiques, et plus particulièrement des cartes test pour analyse dans lesquelles sont aménagés des canaux d'alimentation de fluide jusqu'à des puits adaptés pour recevoir un réactif et un échantillon de fluide ou de test contenant un agent microbiologique tel qu'un microorganisme.

L'objet de l'invention vise plus précisément une technique permettant d'assurer l'obturation de ces puits afin d'éviter tout risque de contamination entre ces différents puits.

Dans l'état de la technique, de nombreuses publications décrivent diverses cartes test pour analyse qui possèdent un puits ou une enceinte de réaction conçu pour recevoir un réactif et un échantillon de fluide contenant un agent microbiologique tel qu'un microorganisme. Une telle carte test qui est par exemple décrite par le brevet EP 0 908 728, comporte une plaque dans laquelle sont aménagés des puits communiquant avec un port d'admission, par l'intermédiaire d'un réseau de canaux d'alimentation aménagés sur l'une et/ou l'autre des faces principales de la plaque.

De manière classique, dans chacun des puits un réactif est déposé lors de la fabrication de la carte test. Le réactif contient habituellement un milieu de culture pour un agent microbiologique contenu dans un échantillon de fluide ou de test. Il est connu ainsi de déposer un réactif différent dans chacun des puits de la carte pour effectuer des tests d'identification sur un échantillon de fluide contenant un agent microbiologique ou un organisme inconnu. Il est aussi connu d'utiliser les cartes pour tester la sensibilité de l'agent microbiologique aux antibiotiques en déposant divers réactifs antibiotiques dans les puits.

Dans l'état de la technique, il est connu d'appliquer une membrane adhésive transparente sur l'une et/ou l'autre des surfaces de la plaque de manière à recouvrir les puits et les canaux d'alimentation de fluide. Cette membrane adhésive transparente empêche que le réactif soit délogé du puits pendant le transport et la manipulation et sert également de barrière de liquide pour empêcher l'échantillon de fluide introduit dans le puits, de fuir sur les bords du puits.

Il doit être considéré qu'il existe une réelle possibilité de communication fluidique entre les puits à échantillon durant les diverses étapes de traitement de la carte test, ce qui est susceptible de provoquer une contamination croisée entre les différents puits. Un tel risque est d'autant plus important que la distance entre les puits a tendance à diminuer en raison de la nécessité d'aménager un plus grand nombre de puits sur une carte test dont les dimensions restent identiques.

Pour réduire le risque de contamination croisée, l'art antérieur a proposé diverses solutions. Par exemple, le brevet US 6 128 899 propose de remplir les différents canaux d'alimentation par l'intermédiaire d'un fluide additionnel afin de séparer les puits entre eux. La demande de brevet US 2008/0112855 propose d'isoler les puits entre eux en interposant un obturateur sensible à la chaleur permettant d'ouvrir ou de fermer les canaux d'alimentation.

Il ressort que ces techniques pour éviter la contamination entre les puits à échantillon nécessitent de modifier le procédé de fabrication des cartes test en intégrant des moyens d'obturation sur la carte. Ces techniques s'avèrent difficiles à mettre en oeuvre en pratique.

La demande de brevet US 2004/0157343 propose de fermer les puits à échantillon en déformant localement la carte test au droit des canaux d'alimentation. La déformation de la carte test est assurée par l'intermédiaire d'un poinçon chauffé. En pratique, cette technique est délicate à mener à bien dans la mesure où il apparaît difficile de contrôler la température et la déformation de la carte test. Il s'ensuit un risque important de dégradation des puits à échantillon, susceptible de compromettre l'intégrité du processus de test des échantillons microbiologiques.

Dans le même sens, la demande de brevet WO 2004/011149 décrit un procédé et un dispositif pour réaliser des obturateurs dans un circuit aménagé sur un substrat. Le circuit comporte une première et une deuxième cavités reliées entre elles par un canal réalisé en un matériau déformable élastiquement. Le substrat est recouvert par une membrane adhésive déformable élastiquement et recouvrant au moins le canal afin de délimiter une communication entre les première et deuxième cavités lorsque la membrane est dans un état non déformé. Le procédé prévoit de déformer à l'aide d'outils spécifiques à la fois la membrane et le matériau constitutif du canal afin de réaliser un obturateur. Selon une variante de réalisation, il est prévu de déformer la membrane afin d'amener la couche d'adhésif en contact avec le canal pour assurer l'obturation entre la première et la deuxième cavités.

Cette technique enseigne d'utiliser un substrat déformable et une membrane adhésive déformable élastiquement, ce qui présente un risque important de dégradation des puits à échantillon. Par ailleurs cette technique ne permet pas d'assurer à coup sûr l'isolation des puits à échantillon.

La présente invention vise donc à remédier aux inconvénients de l'état de la technique en proposant une nouvelle méthode pour isoler les puits à échantillon d'une carte test pour analyses, ce nouveau procédé étant efficace et simple à mettre en oeuvre, tout en permettant de conserver les techniques classiques de fabrication de cartes test pour analyses.

Pour atteindre un tel objectif, l'objet de l'invention concerne un procédé pour isoler au moins un puits à échantillon de fluide aménagé dans une carte test pour analyses, ladite carte test comportant une plaque présentant au moins une première face principale à partir de laquelle est aménagé au moins un canal communiquant avec au moins ledit puits, cette première face étant recouverte par une membrane pourvue d'un adhésif et venant recouvrir ledit canal par une zone de recouvrement, le procédé pour isoler comportant :
- une phase d'introduction d'un échantillon de fluide dans au moins ledit puits,
- une phase pour isoler ledit puits, selon une zone d'isolation, consistant à exercer un effort sur au moins une partie de la zone de recouvrement de la membrane adhésive pour la déplacer jusqu'à l'amener à épouser la forme de la paroi du canal de manière à permettre l'adhésion de ladite zone de recouvrement de la membrane adhésive sur la paroi du canal. Selon l'invention, le procédé consiste à exercer un effort sur la zone de recouvrement de la membrane adhésive afin d'assurer le déplacement de l'adhésif dans une zone d'obturation pour le puits à échantillon contiguë à la zone d'isolation.

Le procédé selon l'invention comporte en combinaison l'une et/ou l'autre des caractéristiques suivantes :
- exercer l'effort sur la zone de recouvrement de la membrane adhésive située en surplomb d'au moins un canal pour assurer le déplacement d'un volume d'adhésif supérieur au volume de la zone d'obturation du canal,
- exercer l'effort sur la zone de recouvrement de la membrane adhésive située en surplomb d'au moins un canal de distribution, afin d'assurer le déplacement de l'adhésif dans au moins une zone d'obturation située dans un canal de remplissage communiquant entre un puits à échantillon et le canal de distribution,
- exercer l'effort sur la zone de recouvrement de la membrane adhésive lors d'un déplacement relatif entre la carte test et l'organe d'appui selon une direction parallèle à la direction d'extension d'au moins un canal de distribution afin d'assurer le déplacement de l'adhésif dans un canal de remplissage communiquant avec ledit canal de distribution,
- exercer un effort sur zone de recouvrement de la membrane adhésive à l'aide d'un organe d'appui présentant une extrémité d'appui de section complémentaire à la section du canal,
- exercer un effort sur la zone de recouvrement de la membrane adhésive selon une pression comprise entre 0,3 et 0,7 Kg/cm² et de préférence de l'ordre de 0,6 Kg/cm²,
- déplacer la carte test selon une direction correspondant au mouvement de translation d'un système de déplacement de la carte test faisant partie d'une machine de traitement de carte test.

Un autre objet de l'invention est de proposer une nouvelle carte test pour analyses, de conception simple et offrant une isolation complète entre les puits à échantillon tout conservant l'intégrité des puits à échantillon.

Pour atteindre un tel objectif, l'objet de l'invention concerne une plaque présentant au moins une face principale à partir de laquelle est aménagé au moins un canal communiquant avec au moins un puits à échantillon, cette première face étant revêtue par une membrane avec adhésif pour recouvrir ledit canal par une zone de recouvrement, au moins une partie de la zone de recouvrement de la membrane adhésive épouse au moins localement la forme de la paroi du canal, dans une zone d'isolation. Selon l'invention, la carte test comporte au moins une zone d'obturation pour le puits à échantillon, adjacente à la zone d'isolation et remplie par l'adhésif de la membrane adhésive issu de la zone d'obturation.

De manière avantageuse, la carte test selon l'invention comporte en combinaison l'une et/ou l'autre des caractéristiques additionnelles suivantes :
- au moins une zone d'obturation située dans un canal de distribution, au moins à l'intersection avec au moins un canal de remplissage pour un puits à échantillon, la zone d'isolation adjacente à la zone d'obturation étant située dans le canal de remplissage,
- la membrane adhésive est revêtue d'un adhésif présentant une épaisseur comprise entre 15 et 100 µm,
- le canal de distribution ou de remplissage possède une section comprise entre d'une part un demi-cercle de hauteur R et d'une largeur égale à 2R et d'autre part, une portion de cercle de largeur √ 5.R/3 et d'une hauteur égale à R/3.

Un autre objet de l'invention est de proposer un dispositif d'obturation pour la mise en oeuvre du procédé conforme à l'invention.

Selon cet aspect de l'invention, le dispositif d'obturation pour la mise en oeuvre du procédé pour isoler au moins un puits à échantillon aménagé dans une carte test pour analyse comporte un système pour exercer un effort sur au moins une partie de la zone de recouvrement de la membrane adhésive pour la déplacer jusqu'à l'amener à épouser au moins localement la forme de la paroi du canal de manière à isoler ledit puits par rapport au canal en chassant l'adhésif par application de l'effort sur la membrane adhésive.

Avantageusement, le dispositif d'obturation comporte l'une et/ou l'autre des caractéristiques additionnelles suivantes :
- le système pour exercer un effort comporte un organe d'appui de section complémentaire à la section du canal,
- l'organe d'appui possède une extrémité d'appui arrondie.

Selon une première variante de réalisation, l'organe d'appui est piloté en rapprochement/écartement par rapport à la carte test pour permettre d'appliquer au moins une partie de la zone de recouvrement de la membrane adhésive sur la paroi du canal.

Selon une deuxième variante de réalisation, l'organe d'appui est monté mobile en rotation pour rouler sur au moins une partie de la longueur du canal.

Avantageusement, un autre objet de l'invention est de proposer une machine de traitement comportant un dispositif d'obturation conforme à l'invention.

Selon une variante préférée de réalisation, le dispositif d'obturation est monté pour agir sur la carte test lors d'un mouvement de translation de moyens de convoyage faisant partie de la machine de traitement.

Diverses autres caractéristiques ressortent de la description faite ci-dessous en référence aux dessins annexés qui montrent, à titre d'exemples non limitatifs, des formes de réalisation de l'objet de l'invention.

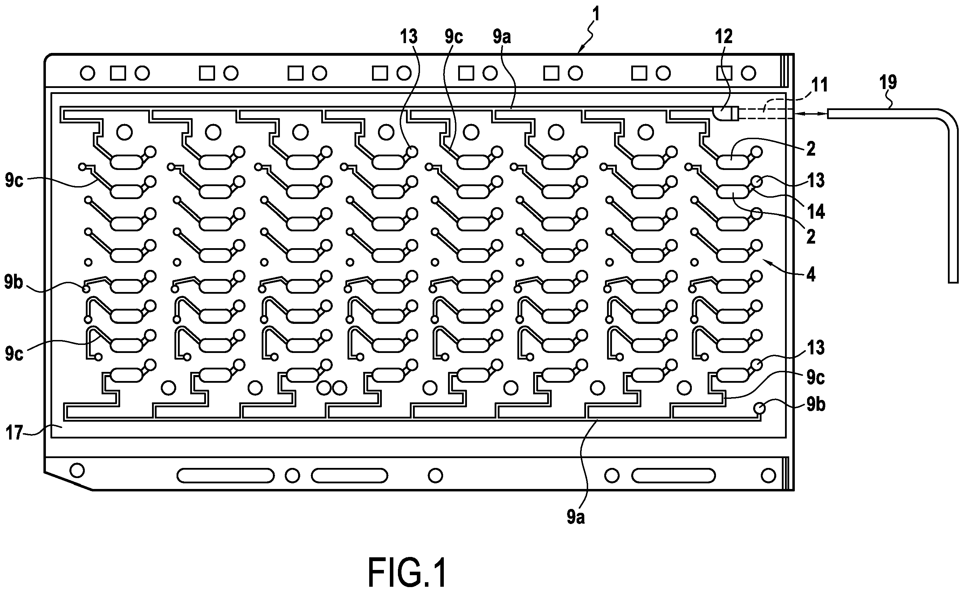

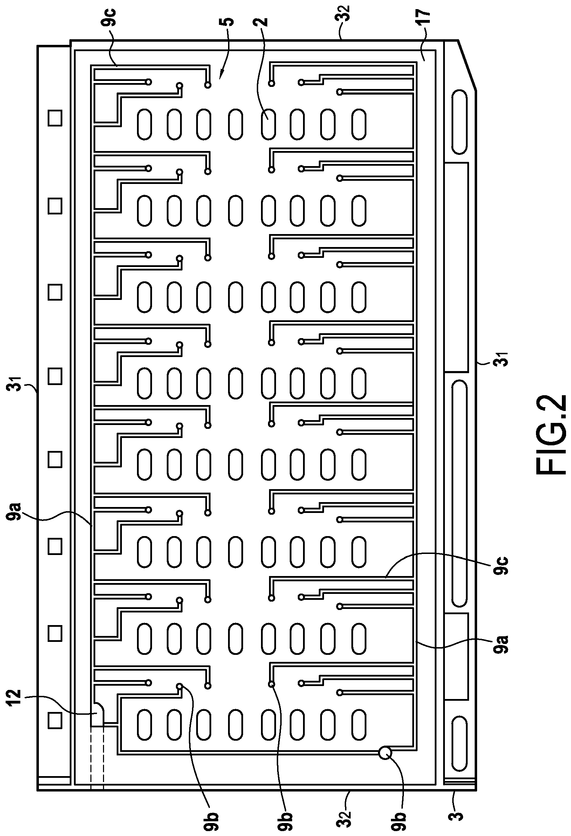

**Les** **Figure 1** et **2** sont des vues en plan respectivement des faces avant et arrière d'un exemple de réalisation d'une carte test mettant en oeuvre le procédé conforme à l'invention.

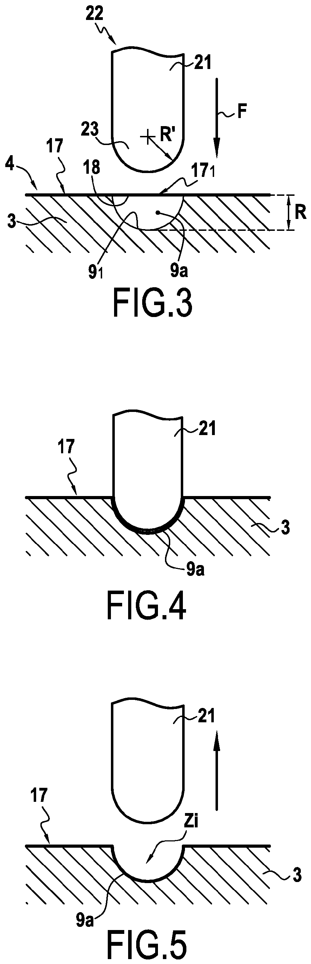

**Les** **Figures 3** à **5** sont des vues en coupe transversale à grande échelle d'une carte test montrant trois étapes caractéristiques du procédé conforme à l'invention, à savoir respectivement, avant l'application d'un effort sur la membrane adhésive, pendant l'application d'un effort sur la membrane adhésive et après la suppression de l'application d'un effort sur la membrane adhésive de la carte test.

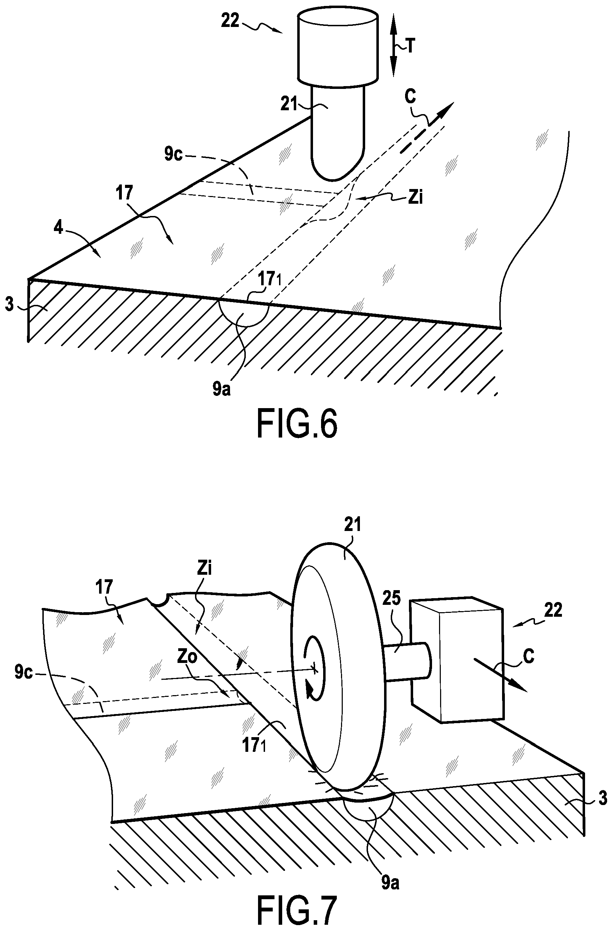

**La** **Figure 6** est une vue schématique d'un exemple de mise en oeuvre du procédé selon l'invention visant à appliquer un effort sur la membrane adhésive de la carte test selon une direction perpendiculaire à la face de la carte test.

**La** **Figure 7** est une vue schématique d'un autre exemple de mise en oeuvre du procédé selon l'invention visant à appliquer un effort sur la membrane adhésive de la carte test, simultanément à un déplacement de l'application de l'effort selon la direction d'extension du canal.

**Les** **Fig. 1** et **2** illustrent un exemple de réalisation d'une carte test **1** comportant des puits à échantillon **2**, isolés entre eux. Avant de décrire les caractéristiques d'obturation des puits **2**, il est rappelé qu'une carte test **1** pour analyse se présente généralement sous la forme d'une plaque ou support **3** de faible épaisseur présentant une première face principale dite avant **4** et une deuxième face principale opposée **5** dite arrière. Dans l'exemple illustré, la plaque **3** possède une forme rectangulaire et présente deux bords longitudinaux **3₁** parallèles entre eux et reliés par deux bords transversaux **3₂** également parallèles entre eux.

Cette carte test **1** comporte au moins un et dans l'exemple illustré, une pluralité de puits **2** agencés dans un mode de réalisation préféré en rangées et en colonnes. Dans l'exemple illustré, les puits **2** répartis en huit rangées et huit colonnes débouchent sur les deux faces **4** et **5.** Bien entendu, le nombre, la géométrie et la disposition des puits **2** peuvent être réalisés de manière différente. Classiquement les puits **2** sont pré-remplis de réactifs et/ou de milieux de culture pour pouvoir réaliser un test biologique sur des échantillons.

La carte test **1** comporte également au moins un et d'une manière générale, un réseau de canaux **9a**, **9b**, **9c** aménagés dans la plaque **3** pour acheminer l'échantillon de fluide à partir d'un port d'admission de fluide **11** jusqu'aux puits **2.** Les canaux d'alimentation ou d'acheminement **9a**, **9b**, **9c** sont aménagés sur l'une et/ou l'autre des faces principales **4**, **5.** Ces canaux d'alimentation peuvent être répartis en des canaux de distribution **9a** reliés au port d'admission **11** et sur lesquels sont raccordés en parallèle, des canaux de remplissage **9c** aboutissant chacun à un puits **2.**

Dans l'exemple illustré, la carte test **1** comporte des canaux de distribution de fluide **9a** aménagés sur les surfaces des faces avant **4** et arrière **5** de la plaque **3.** De manière avantageuse, la partie des canaux de distribution **9a** sur laquelle se raccordent les canaux de remplissage **9c** s'étend selon une direction parallèle aux bords longitudinaux **3₁**. Les canaux de distribution **9a** communiquent entre eux d'une face à l'autre, à l'aide de canaux de liaison **9b** traversant la plaque **3** de part en part. Ces canaux de distribution **9a** communiquent avec le port d'admission **11** par l'intermédiaire d'un collecteur d'admission **12.**

Ces canaux de distribution **9a** sont reliés aux puits **2** par l'intermédiaire des canaux de remplissage **9c** aménagés sur les deux faces **4**, **5** de la plaque. Chaque puits **2** communique ainsi avec un canal de distribution **9a** par l'intermédiaire d'un canal de remplissage **9c** aménagé sur une face de la plaque ou avec deux canaux de remplissage **9c** réalisés sur les deux faces **4**, **5** et communiquant en série à l'aide d'un canal de liaison **9b** traversant de part en part la plaque **3.**

Selon une variante de réalisation, les puits **2** sont en communication avec des pièges à bulles **13** par l'intermédiaire de passages **14** qui permettent de diriger toutes les bulles qui peuvent se former dans le puits **2** jusqu'au piège à bulles correspondant **13.**

Une membrane adhésive **17** est appliquée sur chaque face **3**, **4** de la plaque. La membrane adhésive **17** est de préférence transparente et comporte un support pour un adhésif **18.** Ce support qui possède une épaisseur comprise entre 18 et 100 µm, se trouve réalisé par exemple par un film PMP (polyméthyl-pentène) possédant une épaisseur de l'ordre de 50 µm ou par un film BOPP (polypropylène orienté biaxial) d'épaisseur de l'ordre de 19 µm. Cette membrane adhésive **17** est revêtue classiquement d'un adhésif **18** présentant une épaisseur de l'ordre de 30 µm et de préférence comprise entre 15 et 100 µm.

Cette membrane adhésive **17** présente un caractère déformable élastiquement ou plastiquement. Cette membrane adhésive **17** présente une élongation à la rupture supérieure ou égale à 160 %.

La membrane adhésive **17** recouvre ainsi les puits **2** et les canaux **9a**, **9b**, **9c** pour assurer leur fermeture dans le plan des faces **3**, **4** de la carte. Il doit être noté que lors de l'application de la membrane adhésive **17** sur la plaque **3**, la membrane adhésive **17** adhère aux faces **4**, **5** sans venir adhérer aux parois **9₁** des canaux **9a**, **9b**, **9c** aménagés en creux à partir des faces **4**, **5** de la plaque **3.** Ainsi, la membrane adhésive **17** recouvre en particulier les canaux de distribution **9a** et les canaux de remplissage **9c**, par une zone **17₁** dite de recouvrement. En d'autres termes, cette zone de recouvrement **17₁** correspond à la partie de la membrane adhésive **17** située en face, en surplomb ou en vis-à-vis des canaux de distribution **9a** et des canaux de remplissage **9c**.

Cette phase de fabrication d'une telle carte test **1** n'est pas décrite plus précisément dans la mesure où elle ne fait pas partie précisément de l'objet de l'invention et est bien connue de l'homme du métier. La fabrication d'une telle carte test **1** est par exemple décrite plus en détails dans les brevets US 5 609 928, EP 0 785 433 ou EP 0 745 856. Bien entendu, l'objet de l'invention s'applique à une carte test **1** différente de celle décrite à titre d'exemple aux **Fig. 1** et **2****.**

Lors d'une phase d'utilisation, les puits **2** de la carte test **1** sont remplis par un échantillon de fluide ou de test au moyen de techniques de dépôt sous vide connues. Classiquement, une extrémité d'un tube de transfert **19** est insérée dans le port d'admission du fluide **11** et fixée en place. L'autre extrémité du tube de transfert **19** est placée dans un réceptacle tel qu'un tube à essai contenant l'échantillon de fluide. Le fluide est soutiré par le tube de transfert **19** jusqu'au collecteur d'admission **12** qui alimente les canaux **9a**, **9b**, **9c** assurant l'acheminement du fluide jusqu'aux puits **2.**

Après cette phase d'introduction d'un échantillon de fluide dans les puits **2** de la carte test **1**, l'objet de l'invention vise à mettre en oeuvre une phase pour isoler au moins un et de préférence tous les puits **2** de la carte test **1** afin d'éviter une contamination croisée entre les puits.

Le procédé ou la méthode pour réaliser cette phase d'isolation consiste comme cela apparaît plus particulièrement aux **Fig. 3** à **5**, à exercer un effort **F** sur au moins une partie de la zone de recouvrement **17₁** de la membrane adhésive **17**, située en surplomb ou en face d'au moins un canal qui sur les dessins est un canal de distribution **9a**. Bien entendu, il peut être envisagé d'appliquer un effort sur au moins une partie de la zone de recouvrement **17₁** de la membrane adhésive **17** située en surplomb ou en face d'un canal de remplissage **9c.**

Le procédé selon l'invention consiste donc à déplacer au moins une partie de la zone de recouvrement **17₁** de la membrane adhésive **17**, pour l'amener à épouser la forme de la paroi **9₁** du canal de manière à permettre l'adhésion de cette partie de la zone de recouvrement **17₁** de la membrane adhésive **17** sur la paroi **9₁** du canal (**Fig. 4**). Dans la mesure où cette partie de la zone de recouvrement **17₁** de la membrane adhésive **17** est amenée en contact avec la paroi du canal **9a**, la membrane adhésive **17** est à même d'adhérer ou de rester collée grâce à l'adhésif **18**, à la paroi du canal **9a** dans une zone d'isolation **Zi** (**Fig. 5**). Il est à noter que la membrane adhésive **17** est amenée en contact avec la paroi d'un canal, sans déformation du support **3.**

L'application d'un effort **F** sur la zone de recouvrement **17₁** de la membrane adhésive **17** afin de l'amener à adhérer à la paroi d'un canal peut être assurée par tous moyens techniques appropriés. Dans l'exemple de réalisation illustré aux **Fig. 3** à **5**, la zone de recouvrement **17₁** de la membrane adhésive **17** est déplacée à l'aide d'un organe d'appui **21** faisant partie d'un dispositif d'obturation **22.** L'organe d'appui **21** comporte une extrémité d'appui **23** présentant une section complémentaire à la section du canal.

Dans l'exemple illustré aux **Fig. 3** à **5**, le canal de distribution **9a** présente une section droite demi-circulaire de rayon **R.** Ainsi, le canal de distribution **9a** possède une hauteur ou profondeur **R** et une largeur égale **à 2R.**

L'organe d'appui **21** présente également une extrémité d'appui **23** de section demi-circulaire de rayon **R'.** Bien entendu, l'organe d'appui **21** est placé relativement au canal de manière à obtenir une congruence entre les sections du canal et de l'extrémité d'appui **23.**

Avantageusement, l'extrémité d'appui **23** de l'organe d'appui **21** possède un rayon **R'** égal au rayon **R** du canal, diminué de l'épaisseur de la membrane adhésive **17.** L'organe d'appui **21** assure l'étirement ou la déformation de la membrane adhésive **17** dans sa zone de recouvrement **17₁** afin de l'amener au contact avec la paroi du canal afin de permettre à l'adhésif **18** d'adhérer à la paroi du canal.

Il est à noter que l'organe d'appui **21** présente une extrémité d'appui **23** de forme générale arrondie pour éviter de percer la membrane adhésive **17.** Ainsi, comme cela ressort de la **Fig. 6**, l'organe d'appui **21** possède dans un plan parallèle à la direction d'extension du canal, une section arrondie.

Selon une variante préférée de réalisation, l'organe d'appui **21** exerce sur la membrane adhésive **17**, une pression comprise entre 0,3 et 0,7 kg/cm² et de préférence de l'ordre de 0,6 kg/cm².

Dans les exemples de réalisation illustrés, chaque canal possède une section droite demi-circulaire. Bien entendu, les canaux peuvent présenter des sections de forme différente. Ainsi, il peut être prévu de réaliser un canal avec une section comprise entre d'une part un demi-cercle de hauteur **R** et une largeur **2R** et d'autre part, une portion de cercle de largeur √ 5.R/3 et d'une hauteur égale à R/3. Typiquement, dans le cas d'un canal de section demi-circulaire, le rayon **R** du canal est compris entre 50 µm et 2,5 mm et de préférence entre 150 µm et 500 µm.

Dans l'exemple illustré aux **Fig. 3** à **5**, l'application de l'effort d'appui **F** sur la zone de recouvrement **17₁** de la membrane adhésive **17** est obtenue en assurant un mouvement de rapprochement relatif entre l'organe d'appui **21** et la carte test **1.** Après l'adhésion de la membrane adhésive **17** sur la paroi du canal, l'organe d'appui **21** et la carte test **1** sont écartés l'un de l'autre.

Dans un exemple de réalisation illustré à la **Fig. 6**, l'organe d'appui **21** peut être piloté en rapprochement/écartement par rapport à la carte test **1** à l'aide d'un système de déplacement linéaire alternatif de tous types connus en soi. Selon cette variante, le système de déplacement pilote le mouvement de coulissement de l'organe d'appui **21** selon une direction **T** perpendiculaire à la face **4** ou **5** de la carte test **1.** Selon un autre exemple de réalisation, la carte test **1** peut être déplacée en rapprochement/écartement par rapport à l'organe d'appui **21** qui reste fixe.

Selon une variante avantageuse de réalisation, le procédé selon l'invention vise à exercer l'effort sur la zone de recouvrement **17₁** de la membrane adhésive **17**, simultanément à un déplacement relatif entre la carte test **1** et l'organe d'appui **21** selon une direction parallèle à la direction d'extension du canal afin de permettre l'adhésion de la zone de recouvrement **17₁** de la membrane adhésive **17** sur au moins une partie de la longueur dudit canal, supérieure à l'extrémité d'appui de l'organe d'appui **21.**

Tel que cela ressort de la **Fig. 6**, l'organe d'appui **21** peut posséder en supplément du mouvement de translation **T**, perpendiculaire à la face **4**, **5** de la carte test **1**, un mouvement de coulissement **C** parallèle à la face **4**, **5** de la carte test **1** et selon la direction d'extension du canal c'est-à-dire selon une variante avantageuse de réalisation, la longueur d'un canal de distribution **9a**.

La **Fig. 7** illustre une autre variante de réalisation d'un organe d'appui **21** monté mobile en rotation autour d'un axe de rotation **25** pour rouler sur le fond du canal de distribution **9a** et selon une partie de sa longueur prise selon la direction d'extension **C** du canal de distribution **9a**. Selon cet exemple, l'organe d'appui **21** se présente sous la forme d'un galet dont la circonférence possède une section droite arrondie complémentaire de la section du canal comme indiqué précédemment.

Selon une caractéristique de l'invention, il est à noter que l'application d'un effort **F** sur la membrane adhésive **17** venant en contact avec la paroi du canal conduit à un déplacement de l'adhésif situé dans la zone d'application de cet effort. Il doit être compris que la majeure partie de l'adhésif **18** est déplacée par l'organe d'appui **21**, dans une zone d'obturation **Zo**.

Ainsi, le déplacement de l'organe d'appui **21** selon la direction d'extension **C** des canaux, comme illustré à la **Fig. 7**, conduit concomitamment au déplacement de l'adhésif **18.** En effet, lors du déplacement de l'organe d'appui **21** à l'intérieur du canal, une partie de l'adhésif est chassée ou poussée par l'organe d'appui **21** qui agit comme un organe de marouflage. L'adhésif **18** s'établit donc dans au moins une zone d'obturation **Zo** adjacente à la zone d'isolation **Zi.** Il est à noter que dans cette zone d'isolation **Zi**, la membrane adhésive **17** adhère ou non à la paroi du canal, l'important étant que l'adhésif chassé vienne obturer le canal en amont des puits à échantillon.

Il est à noter qu'il peut être obtenu cet effet de marouflage avec un organe d'appui **21** déplacé uniquement en translation **T** perpendiculaire à une face de la carte test **1.** Dans ce cas, l'adhésif **18** est chassé de part et d'autre de la zone d'application de l'organe d'appui **21**, dans le canal de distribution **9a**, voire dans un canal de remplissage **9c** lorsque la zone d'application de l'effort est située dans ce canal ou à l'intersection entre un canal de distribution **9a** et un canal de remplissage **9c.**

Le procédé selon l'invention consiste à choisir un ou plusieurs endroits de la zone de recouvrement **17₁** de la membrane adhésive **17**, de manière à créer dans un canal, une zone d'isolation **Zi** et par suite une zone d'obturation **Zo** pour au moins un puits **2** et de préférence pour chaque puits **2**, évitant une contamination entre l'ensemble des puits de la carte. Ces zones d'isolation **Zi** pour les puits **2** peuvent être réalisées en tous endroits appropriés des canaux de distribution **9a** et des canaux de remplissage **9c** pour assurer l'isolation des puits entre eux. Ces zones d'isolation **Zi** s'étendent sur une longueur plus ou moins importante de ces canaux.

Selon une première variante de réalisation, il peut être prévu de réaliser une zone d'isolation **Zi** sur chaque canal de remplissage **9c** d'un puits **2.**

Selon une deuxième variante de réalisation, il peut être prévu de réaliser des zones d'isolation **Zi** dans les canaux de distribution **9a**, à l'intersection avec les canaux de remplissage **9c** et/ou entre deux canaux de remplissage **9c** voisins débouchant sur le canal de distribution **9a**.

Selon un premier exemple de réalisation, chaque zone d'isolation **Zi** possède un caractère ponctuel dans le sens où la partie de la zone de recouvrement **17₁** de la membrane adhésive **17** adhérant à la paroi du canal présente une surface limitée par rapport à la longueur du canal. La **Fig. 6** illustre de manière schématique et agrandie, la réalisation d'une zone d'isolation **Zi** à caractère ponctuel, à l'aide d'un organe d'appui **21** de longueur limitée par rapport à la longueur du canal. Le mouvement de déplacement de l'organe d'appui **21** selon la direction **T** permet d'obtenir dans le canal, au droit de l'organe d'appui, une zone d'isolation **Zi.** Un tel mouvement de l'organe d'appui **21** est à même d'entrainer un déplacement concomitant de l'adhésif **18** susceptible de venir obturer un canal de remplissage **9c** qui débouche dans le canal de distribution **9a** au droit de l'application de l'effort d'appui. Bien entendu, il peut être envisagé de réaliser simultanément plusieurs zones d'isolation **Zi** avec autant d'organes d'appui **21** ou successivement avec un ou plusieurs organes d'appui **21** déplacés en divers lieux de la carte test **1.**

Selon un deuxième exemple de réalisation, la zone d'isolation **Zi** possède un caractère étendu. A cet effet, soit l'organe d'appui **21** présente une extrémité d'appui de grande dimension, soit l'organe d'appui **21** est déplacé relativement à la carte test **1** selon la direction d'extension **C** du canal (**Fig. 7**).

Selon une caractéristique de l'invention, le procédé selon l'invention consiste à réaliser une zone d'isolation **Zi** sur la longueur des canaux de distribution **9a** dans laquelle débouchent les canaux de remplissage **9c.** Avantageusement, cette zone d'isolation **Zi** est continue sur toute la longueur sur laquelle débouchent les canaux de remplissage **9c.** Il s'ensuit que tous les puits **2** communiquant avec ces canaux de remplissage **9c** se trouvent isolés les uns des autres.

Selon un aspect avantageux de l'invention, le procédé selon l'invention permet grâce au déplacement de l'organe d'appui **21** à l'intérieur des canaux de distribution **9a**, d'obtenir le déplacement concomitant de l'adhésif **18** dans les canaux de remplissage **9c**, débouchant dans les canaux de distribution **9a**. Ainsi, la carte test **1** comporte une zone d'isolation **Zi** continue sur chaque canal de distribution **9a**, sur toute sa longueur comportant des intersections avec les canaux de remplissage **9c** afin d'assurer leur isolation. De plus, chaque canal de remplissage **9c** comporte une zone d'obturation **Zo** pourvue de l'adhésif **18** qui est contiguë ou adjacente avec la zone d'obturation **Zo** du canal de distribution **9a**. Une telle disposition assure l'isolation inter puits **2** dans la mesure où l'adhésif **18** vient combler une section des canaux de remplissage **9c.**

Il ressort de la description qui précède que quel que soit le mode d'application de l'effort sur la membrane adhésive **17**, dans une zone d'isolation **Zi**, cet effort conduit conformément à l'invention, à un déplacement de la majeure partie du volume de l'adhésif situé dans cette zone d'isolation et sur laquelle agit l'organe d'appui **21.** Avantageusement, l'effort exercé sur la membrane adhésive **17** est adapté pour assurer le déplacement d'un volume d'adhésif **18** supérieur au volume de la zone d'obturation **Zo** du canal **9a**, **9c** afin de garantir l'obturation dudit canal par le volume d'adhésif **18** déplacé. Par exemple, le volume d'adhésif **18** déplacé est le double du volume du canal à obturer afin d'obtenir une fermeture hermétique du canal.

Il ressort de la description qui précède que le procédé selon l'invention est de mise en oeuvre simple puisqu'il nécessite l'application d'un effort sur la zone de recouvrement **17₁** de la membrane adhésive **17** afin de permettre son adhésion sur la paroi **9₁** du canal et le déplacement de l'adhésif **18** chassé à la suite de l'application de cet effort. Le support **3** ne subit pas de déformation lors de l'application de cet effort, permettant de préserver l'intégrité des puits à échantillon. Dans les exemples décrits ci-dessus, l'application de cet effort est assurée par l'intermédiaire d'un organe d'appui **21** de section complémentaire à la section du canal. Bien entendu, le déplacement de la zone de recouvrement **17₁** de la membrane adhésive **17** peut être assuré de manière différente. Par exemple, il peut être envisagé d'appliquer l'effort d'appui sur la zone de recouvrement **17₁** de la membrane adhésive **17** à l'aide d'un jet d'air.

Selon une variante avantageuse, le dispositif d'obturation **22** est mis en oeuvre dans le cadre d'une machine de traitement pour des cartes test **1** telle que décrite par exemple par les brevets US 7 601 300, US 5 798 085, US 5 853 666 ou US 5 888 455.

De manière classique, une machine de traitement pour des cartes test comporte une succession de postes de traitement à savoir notamment un poste de chargement, un poste d'identification des cartes test, un poste pour étancher la carte test par rapport à l'environnement extérieur, un poste d'incubation et un poste de lecture des cartes test. Bien entendu, une telle machine comporte des moyens de convoyage permettant le déplacement des cartes test à l'intérieur des postes et d'un poste à l'autre.

Avantageusement, la phase pour isoler les puits **2** à l'aide du procédé conforme à l'invention est réalisée lors d'un mouvement de translation des cartes test **1**, effectué juste après le remplissage par l'échantillon à tester et préalablement au poste de lecture. Ainsi, lors du déplacement des cartes test **1**, le mouvement de translation des cartes test est utilisé pour permettre lors d'une partie de ce mouvement de translation, l'application de l'organe d'appui **21** sur la zone de recouvrement **17₁** de la membrane adhésive **17** pour permettre son adhésion sur la paroi du canal de distribution **9a**. Ainsi comme le mouvement de translation des cartes test **1** est parallèle à la direction d'extension des canaux de distribution **9a**, l'organe d'appui **21** peut rouler sur le fond du canal de distribution **9a** selon tout ou partie de sa longueur. Il s'ensuit que la phase d'obturation des puits **2** peut être réalisée en temps masqué puisqu'elle peut intervenir lors d'un mouvement de translation des cartes test **1** effectué par n'importe quels moyens de convoyage à déplacement linéaire faisant partie de la machine de traitement. Ainsi, l'obturation des puits **2** est obtenue simplement en équipant la machine de traitement d'un dispositif d'obturation **22** conforme à l'invention à proximité de moyens de convoyage faisant partie de la machine de traitement et assurant un mouvement de translation aux cartes test **1.**

## Revendications

1. Procédé pour isoler au moins un puits (**2**) à échantillon de fluide aménagé dans une carte test (**1**) pour analyses, ladite carte test (**1**) comportant une plaque (**3**) présentant au moins une première face principale à partir de laquelle est aménagé au moins un canal (**9a**, **9b**, **9c**) communiquant avec au moins ledit puits, cette première face étant revêtue par une membrane (**17**) pourvue d'un adhésif (**18**) et venant recouvrir ledit canal (**9a**, **9c**) par une zone de recouvrement (**17₁**), le procédé pour isoler comportant :
- une phase d'introduction d'un échantillon de fluide dans au moins ledit puits (**2**),
- une phase pour isoler ledit puits (**2**), selon une zone d'isolation (**Zi**), consistant à exercer un effort sur au moins une partie de la zone de recouvrement (**17₁**) de la membrane adhésive (**17**) pour la déplacer jusqu'à l'amener à épouser la forme de la paroi (**9₁**) du canal (**9a**, **9c**) de manière à permettre l'adhésion de ladite zone de recouvrement (**17₁**) de la membrane adhésive (**17**) sur la paroi (**9₁**) du canal, **caractérisé en ce qu'**il consiste à exercer un effort sur la zone de recouvrement (**17₁**) de la membrane adhésive (**17**) afin d'assurer le déplacement de l'adhésif (**18**) dans une zone d'obturation (**Zo**) pour le puits à échantillon, contigüe à la zone d'isolation (**Zi**).

2. Procédé selon la revendication 1, **caractérisé en ce qu'**il consiste à exercer l'effort sur la zone de recouvrement (**17₁**) de la membrane adhésive (**17**) située en surplomb d'au moins un canal (**9a**, **9c**) pour assurer le déplacement d'un volume d'adhésif (**18**) supérieur au volume de la zone d'obturation (**20**) du canal (**9a**, **9c**).

3. Procédé selon la revendication 1 ou 2, **caractérisé en ce qu'**il consiste à exercer l'effort sur la zone de recouvrement (**17₁**) de la membrane adhésive (**17**) située en surplomb d'au moins un canal de distribution (**9a**), afin d'assurer le déplacement de l'adhésif (**18**) dans au moins une zone d'obturation (**Zo**) située dans un canal de remplissage (**9c**) communiquant entre un puits à échantillon (**2**) et le canal de distribution (**9a**).

4. Procédé selon l'une des revendications 1 à 3, **caractérisé en ce qu'**il consiste à exercer l'effort sur la zone de recouvrement (**17₁**) de la membrane adhésive (**17**) lors d'un déplacement relatif entre la carte test (**1**) et l'organe d'appui (**21**) selon une direction parallèle à la direction d'extension d'au moins un canal de distribution (**9a**) afin d'assurer le déplacement de l'adhésif (**18**) dans un canal de remplissage (**9c**) communiquant avec ledit canal de distribution (**9a**).

5. Procédé selon l'une des revendications 1 à 4, **caractérisé en ce qu'**il consiste à exercer un effort sur zone de recouvrement (**17₁**) de la membrane adhésive (**17**) à l'aide d'un organe d'appui (**21**) présentant une extrémité d'appui (**23**) de section complémentaire à la section du canal.

6. Procédé selon l'une des revendications 1 à 5, **caractérisé en ce qu'**il consiste à exercer un effort sur la zone de recouvrement (**17₁**) de la membrane adhésive (**17**) selon une pression comprise entre 0,3 et 0,7 Kg/cm² et de préférence de l'ordre de 0,6 Kg/cm².

7. Procédé selon la revendication 6 **caractérisé en ce qu'**il consiste à déplacer la carte test (**1**) selon une direction correspondant au mouvement de translation d'un système de déplacement de la carte test faisant partie d'une machine de traitement de carte test.

8. Carte test pour analyses, comportant une plaque (**3**) présentant au moins une première face principale (**4**, **5**) à partir de laquelle est aménagé au moins un canal (**9a**, **9b**, **9c**) communiquant avec au moins un puits à échantillon (**2**), cette première face (**4**) étant revêtue par une membrane (**17**) avec adhésif (**18**) qui recouvre ledit canal (**9a**, **9c**), par une zone de recouvrement (**17₁**), au moins une partie de la zone de recouvrement (**17₁**) de la membrane adhésive (**17**) épouse au moins localement la forme de la paroi (**9₁**) du canal (**9a**, **9c**), dans une zone d'isolation (**Zi**), **caractérisée en ce qu'**elle comporte au moins une zone d'obturation (**Zo**) pour le puits à échantillon (**2**), adjacente à la zone d'isolation (**Zi**) et remplie par l'adhésif (**18**) de la membrane adhésive (**17**), issu de la zone d'isolation.

9. Carte test selon la revendication 8, **caractérisée en ce qu'**elle comporte au moins une zone d'obturation (**Zo**) située dans un canal de distribution (**9a**), au moins à l'intersection avec au moins un canal de remplissage (**9c**) pour un puits à échantillon (**2**), la zone d'isolation (**Zi**) adjacente à la zone d'obturation (**Zo**) étant située dans le canal de remplissage (**9c**).

10. Carte test selon la revendication 9, **caractérisée en ce que** la membrane adhésive (**17**) est revêtue d'un adhésif (**18**) présentant une épaisseur comprise entre 15 et 100 µm.

11. Carte test selon la revendication 10, **caractérisée en ce que** le canal de distribution (**9a**) ou de remplissage (**9c**) possède une section comprise entre d'une part un demi-cercle de hauteur R et d'une largeur égale à 2R et d'autre part, une portion de cercle de largeur √ 5.R/3 et d'une hauteur égale à R/3.

12. Dispositif d'obturation pour la mise en oeuvre du procédé pour isoler conformément à l'une des revendications 1 à 7, au moins un puits à échantillon (**2**) aménagé dans une carte test (**1**) pour analyses, ladite carte test comportant un support (**3**) présentant au moins une première face principale (**4**, **5**) à partir de laquelle est aménagé au moins un canal (**9a**, **9b**, **9c**) communiquant avec au moins ledit puits (**2**), cette première face (**4**, **5**) étant revêtue par une membrane (**17**) pourvue d'un adhésif (**18**) et venant recouvrir ledit canal (**9a**, **9c**), par une zone de recouvrement (**17₁**), **caractérisée en ce que** le dispositif comporte un système (**22**) pour exercer un effort sur au moins une partie de la zone de recouvrement (**17₁**) de la membrane adhésive (**17**) pour la déplacer jusqu'à l'amener à épouser au moins localement la forme de la paroi (**9₁**) du canal (**9a**, **9c**) de manière à isoler ledit puits (**2**) par rapport au canal en chassant l'adhésif par l'application de l'effort sur la membrane adhésive.

13. Dispositif d'obturation selon la revendication 12, **caractérisée en ce que** le système (**22**) pour exercer un effort comporte un organe d'appui (**21**) de section complémentaire à la section du canal.

14. Dispositif d'obturation selon la revendication 13, **caractérisé en ce que** l'organe d'appui (**21**) possède une extrémité d'appui arrondie.

15. Dispositif d'obturation selon la revendication 12, **caractérisé en ce que** l'organe d'appui (**21**) est piloté en rapprochement/écartement par rapport à la carte test (**1**) pour permettre d'appliquer au moins une partie de la zone de recouvrement (**17₁**) de la membrane adhésive (**17**) sur la paroi (**9₁**) du canal.

16. Dispositif d'obturation selon la revendication 13, **caractérisé en ce que** l'organe d'appui (**21**) est monté mobile en rotation pour rouler sur au moins une partie de la longueur du canal (**9a**, **9c**).

17. Machine de traitement de cartes de tests, **caractérisée en ce qu'**elle comporte un dispositif d'obturation (**22**) conforme à l'une des revendications 12 à 16.

18. Machine de traitement selon la revendication 17, **caractérisée en ce que** le dispositif d'obturation (**22**) est monté pour agir sur la carte test (**1**) lors d'un mouvement de translation de moyens de convoyage faisant partie de la machine de traitement.

## Patentansprüche

1. Verfahren zum Isolieren mindestens einer Mulde (2) mit einer Flüssigkeitsprobe, die in einer Testkarte (1) für Analysen angeordnet ist, wobei die Testkarte (1) eine Platte (3) mit mindestens einer ersten Hauptfläche aufweist, von der ausgehend mindestens ein Kanal (9a, 9b, 9c), der mit mindestens der Mulde in Verbindung steht, angeordnet ist, wobei diese erste Fläche mit einer Folie (17) überzogen ist, die mit einem Klebstoff (18) versehen ist und den Kanal (9a, 9c) durch einen Überlappungsbereich (17₁) bedeckt, wobei das Verfahren zum Isolieren umfasst:
- eine Phase des Einführens einer Flüssigkeitsprobe in mindestens die Mulde (2),
- eine Phase zum Isolieren der Mulde (2), entlang einem Isolationsbereich (Zi), die darin besteht, dass eine Kraft auf mindestens einen Teil des Überlappungsbereichs (17₁) der Klebefolie (17) ausgeübt wird, um sie zu bewegen, bis sie gezwungen ist, sich der Form der Wand (9₁) des Kanals (9a, 9c) anzupassen, sodass ein Anhaften des Überlappungsbereichs (17₁) der Klebefolie (17) an der Wand (9₁) des Kanals ermöglicht wird, **dadurch gekennzeichnet, dass** es darin besteht, eine Kraft auf den Überlappungsbereich (17₁) der Klebefolie (17) auszuüben, um die Verschiebung des Klebstoffs (18) in einen Verschlussbereich (Zo) für die Probenmulde zu gewährleisten, der an den Isolationsbereich (Zi) angrenzt.

2. Verfahren gemäß Anspruch 1, **dadurch gekennzeichnet, dass** es darin besteht, die Kraft auf den Überlappungsbereich (17₁) der Klebefolie (17) auszuüben, der mindestens einen Kanal (9a, 9c) überragt, um die Verschiebung eines Volumens an Klebstoff (18) zu gewährleisten, das größer ist als das Volumen des Verschlussbereichs (20) des Kanals (9a, 9c).

3. Verfahren gemäß Anspruch 1 oder 2, **dadurch gekennzeichnet, dass** es darin besteht, die Kraft auf den Überlappungsbereich (17₁) der Klebefolie (17) auszuüben, der mindestens einen Verteilerkanal (9a) überragt, um die Verschiebung des Klebstoffs (18) in mindestens einen Verschlussbereich (Zo) zu gewährleisten, der sich in einem Füllkanal (9c) befindet, der eine Verbindung zwischen einer Probenmulde (2) und dem Verteilerkanal (9a) herstellt.

4. Verfahren gemäß einem der Ansprüche 1 bis 3, **dadurch gekennzeichnet, dass** es darin besteht, die Kraft auf den überlappenden Bereich (17₁) der Klebefolie (17) während einer Relativbewegung zwischen der Testkarte (1) und dem Andruckelement (21) in einer Richtung parallel zur Ausdehnungsrichtung mindestens eines Verteilerkanals (9a) auszuüben, um die Verschiebung des Klebstoffs (18) in einen Füllkanal (9c) zu gewährleisten, der mit dem Verteilerkanal (9a) in Verbindung steht.

5. Verfahren gemäß einem der Ansprüche 1 bis 4, **dadurch gekennzeichnet, dass** es darin besteht, eine Kraft auf den Überlappungsbereich (17₁) der Klebefolie (17) mittels eines Andruckelements (21) auszuüben, das ein Andruckende (23) mit einem zu dem Querschnitt des Kanals komplementären Querschnitt aufweist.

6. Verfahren gemäß einem der Ansprüche 1 bis 5, **dadurch gekennzeichnet, dass** es darin besteht, eine Kraft auf den Überlappungsbereich (17₁) der Klebefolie (17) bei einem Druck zwischen 0,3 und 0,7 kg/cm² und vorzugsweise in der Größenordnung von 0,6 kg/cm² auszuüben.

7. Verfahren gemäß Anspruch 6, **dadurch gekennzeichnet, dass** es darin besteht, die Testkarte (1) in eine Richtung zu bewegen, die der Translationsbewegung eines Testkarten-Verschiebesystems entspricht, das Teil einer TestkartenVerarbeitungsmaschine ist.

8. Testkarte für Analysen, umfassend eine Platte (3) mit mindestens einer ersten Hauptfläche (4, 5), von der ausgehend mindestens ein Kanal (9a, 9b, 9c), der mit mindestens einer Probenmulde (2) in Verbindung steht, angeordnet ist, wobei diese erste Fläche (4) mit einer Folie (17) mit Klebstoff (18) überzogen ist, die den Kanal (9a, 9c) durch einen Überlappungsbereich (17₁) bedeckt, und mindestens ein Teil des Überlappungsbereichs (17₁) der Klebefolie (17) in einem Isolationsbereich (Zi) zumindest lokal an die Form der Wand (9₁) des Kanals (9a, 9c) angepasst ist, **dadurch gekennzeichnet, dass** sie mindestens einen Verschlussbereich (Zo) für die Probenmulde (2) umfasst, der an den Isolationsbereich (Zi) angrenzt und mit dem Klebstoff (18) der Klebefolie (17) aus dem Isolationsbereich gefüllt ist.

9. Testkarte gemäß Anspruch 8, **dadurch gekennzeichnet, dass** sie mindestens einen Verschlussbereich (Zo) umfasst, der sich in einem Verteilerkanal (9a) befindet und zumindest mit mindestens einem Füllkanal (9c) für eine Probenmulde (2) gekreuzt ist, wobei der Isolationsbereich (Zi), der an den Verschlussbereich (Zo) angrenzt, in dem Füllkanal (9c) angeordnet ist.

10. Testkarte gemäß Anspruch 9, **dadurch gekennzeichnet, dass** die Klebefolie (17) mit einem Klebstoff (18) einer Dicke zwischen 15 und 100 µm überzogen ist.

11. Testkarte gemäß Anspruch 10, **dadurch gekennzeichnet, dass** der Verteilerkanal (9a) oder der Füllkanal (9c) einen Querschnitt aufweist, der zwischen einerseits einem Halbkreis mit einer Höhe R und einer Breite von 2R und andererseits einem Kreisabschnitt mit einer Breite von √ 5.R/3 und einer Höhe von R/3 liegt.

12. Verschlussvorrichtung zur Durchführung des Verfahrens zum Isolieren mindestens einer Probenmulde (2), die in einer Testkarte (1) für Analysen angeordnet ist, gemäß einem der Ansprüche 1 bis 7, wobei die Testkarte einen Träger (3) mit mindestens einer ersten Hauptfläche (4, 5) aufweist, von der ausgehend mindestens ein Kanal (9a, 9b, 9c), der mit mindestens der Mulde (2) in Verbindung steht, angeordnet ist, wobei diese erste Fläche (4, 5) mit einer Folie (17) überzogen ist, die mit Klebstoff (18) versehenen ist und den Kanal (9a, 9c) durch einen Überlappungsbereich (17₁) bedeckt, **dadurch gekennzeichnet, dass** die Vorrichtung ein System (22) umfasst, um eine Kraft auf mindestens einen Teil des Überlappungsbereichs (17₁) der Klebefolie (17) auszuüben, um sie zu bewegen, bis sie gezwungen ist, sich zumindest lokal der Form der Wand (9₁) des Kanals (9a, 9c) anzupassen, sodass die Mulde (2) vom Kanal isoliert wird, indem der Klebstoff durch Aufbringen der Kraft auf die Klebefolie verdrängt wird.

13. Verschlussvorrichtung gemäß Anspruch 12, **dadurch gekennzeichnet, dass** das System (22) zum Ausüben einer Kraft ein Andruckelement (21) mit einem zu dem Querschnitt des Kanals komplementären Querschnitt aufweist.

14. Verschlussvorrichtung gemäß Anspruch 13, **dadurch gekennzeichnet, dass** das Andruckelement (21) ein abgerundetes Andruckende aufweist.

15. Verschlussvorrichtung gemäß Anspruch 12, **dadurch gekennzeichnet, dass** eine Annäherung bzw. Entfernung des Andruckelements (21) gegenüber der Testplatte (1) gesteuert wird, um zu ermöglichen, dass zumindest ein Teil des Überlappungsbereichs (17₁) der Klebefolie (17) auf die Wand (9₁) des Kanals aufgebracht wird.

16. Verschlussvorrichtung gemäß Anspruch 13, **dadurch gekennzeichnet, dass** das Andruckelement (21) drehbar montiert ist, um über mindestens einen Teil der Länge des Kanals (9a, 9c) zu rollen.

17. Testkartenverarbeitungsmaschine, **dadurch gekennzeichnet, dass** sie eine Verschlussvorrichtung (22) entsprechend einem der Ansprüche 12 bis 16 aufweist.

18. Verarbeitungsmaschine gemäß Anspruch 17, **dadurch gekennzeichnet, dass** die Verschlussvorrichtung (22) so montiert ist, dass sie während einer Translationsbewegung von Fördermitteln, die Teil der Verarbeitungsmaschine sind, auf die Testkarte (1) wirkt.

## Claims

1. A method for isolating at least one fluid sample well (2) laid out in a test card (1) for analysis, said test card (1) including a plate (3) having at least one first main face from which is laid out at least one channel (9a, 9b, 9c) communicating with at least said well, this first face being coated with a membrane (17) provided with an adhesive (18) and which covers said channel (9a, 9c) with a covering area (17₁), the method for isolation including:
- a phase for introducing a fluid sample into at least said well (2),
- a phase for isolating said well (2), along an isolation area (Zi), consisting of exerting a force on at least one portion of the covering area (17₁) of the adhesive membrane (17) for displacing it until causing it to fit the shape of the wall (9₁) of the channel (9a, 9c) so as to allow adhesion of said covering area (17₁) of the adhesive membrane (17) onto the wall (9₁) of the channel, **characterized in that** it consists of exerting a force on the covering area (17₁) of the adhesive membrane (17) in order to ensure displacement of the adhesive (18) in an obturation area (Zo) for the sample well, contiguous to the isolation area (Zi).

2. The method according to claim 1, **characterized in that** it consists of exerting the force on the covering area (17₁) of the adhesive membrane (17) located overhanging at least one channel (9a, 9c) for ensuring the displacement of a volume of adhesive (18) greater than the volume of the obturation area (20) of the channel (9a, 9c).

3. The method according to claim 1 or 2, **characterized in that** it consists of exerting the force on the covering area (17₁) of the adhesive membrane (17) located overhanging at least one distribution channel (9a), in order to ensure displacement of the adhesive (18) in at least one obturation area (Zo) located in a filling channel (9c) communicating between a sample well (2) and the distribution channel (9a).

4. The method according to one of claims 1 to 3, **characterized in that** it consists of exerting the force on the covering area (17₁) of the adhesive membrane (17) during a relative displacement between the test card (1) and the supporting member (21) along a direction parallel to the extension direction of at least one distribution channel (9a) in order to ensure the displacement of the adhesive (18) in a filling channel (9c) communicating with said distribution channel (9a).

5. The method according to one of claims 1 to 4, **characterized in that** it consists of exerting a force on the covering area (17₁) of the adhesive membrane (17) by means of a supporting member (21) having a supporting end (23) with a section mating the section of the channel.

6. The method according to one of claims 1 to 5, **characterized in that** it consists of exerting a force on the covering area (17₁) of the adhesive membrane (17) according to a pressure comprised between 0.3 and 0.7 kg/cm² and preferably of the order of 0.6 kg/cm².

7. The method according to claim 6, **characterized in that** it consists of displacing the test card (1) along a direction corresponding to the translational movement of a system for displacing the test card belonging to a test card processing machine.

8. A test card for analyses, including a plate (3) having at least one main face (4, 5) from which is laid out at least one channel (9a, 9b, 9c) communicating with at least one sample well (2), this first face (4) being coated with a membrane (17) having an adhesive (18) which covers said channel (9a, 9c), with a covering area (17₁), at least one portion of the covering area (17₁) of the adhesive membrane (17) at least locally fits the shape of the wall (9₁) of the channel (9a, 9c), in an isolation area (Zi), **characterized in that** it includes at least one obturation area (Zo) for the sample well (2), adjacent to the isolation area (Zi) and filled with the adhesive (18) of the adhesive membrane (17), stemming from the isolation area.

9. The test card according to claim 8, **characterized in that** it includes at least one obturation area (Zo) located in a distribution channel (9a), at least at the intersection with at least one filling channel (9c) for a sample well (2), the isolation area (Zi) adjacent to the obturation area (Zo) being located in the filling channel (9c).

10. The test card according to claim 9, **characterized in that** the adhesive membrane (17) is coated with an adhesive (18) having a thickness comprised between 15 and 100 µm.

11. The test card according to claim 10, **characterized in that** the distribution (9a) or filling (9c) channel has a section comprised between a semi-circle of height R and with a width equal to 2R on the one hand, and a circular portion of width √5·R/3 and with a height equal to R/3 on the other hand.

12. An obturation device for applying the method for isolating, according to one of claims 1 to 7, at least one sample well (2) laid out in a test card (1) for analysis, said test card including a support (3) having at least one first main face (4, 5) from which is laid out at least one channel (9a, 9b, 9c) communicating with at least said well (2), this first face (4, 5) being coated with a membrane (17) provided with an adhesive (18) and which covers said channel (9a, 9c), with a covering area (17₁), **characterized in that** the device includes a system (22) for exerting a force on at least one portion of the covering area (17₁) of the adhesive membrane (17) for displacing it until causing it to at least locally fit the shape of the wall (9₁) of the channel (9a, 9c) so as to isolate said well (2) with respect to the channel by driving out the adhesive by applying the force on the adhesive membrane.

13. The obturation device according to claim 12, **characterized in that** the system (22) for exerting a force includes a supporting member (21) with a section mating the section of the channel.

14. The obturation device according to claim 13, **characterized in that** the supporting member (21) has a rounded supporting end.

15. The obturation device according to claim 12, **characterized in that** the supporting member (21) is driven for moving closer/moving away relatively to the test card (1) in order to allow application of at least one portion of the covering area (17₁) of the adhesive membrane (17) on the wall (9₁) of the channel.

16. The obturation device according to claim 13, **characterized in that** the supporting member (21) is mounted so as to be mobile in rotation in order to roll on at least one portion of the length of the channel (9a, 9c).

17. A machine for processing test cards, **characterized in that** it includes an obturation device (22) according to one of claims 12 to 16.

18. The processing machine according to claim 17, **characterized in that** the obturation device (22) is mounted for acting on the test card (1) during a translational movement of conveying means belonging to the processing machine.
